# EUROPÄISCHE PATENTANMELDUNG

(11) **EP 0 867 423 A1**
(43) Veröffentlichungstag der Anmeldung: **30.09.1998**
(21) Anmeldenummer: 98105394.5
(22) Anmeldetag: 25.03.1998
(51) Int. Cl.: C07C 15/16, C07C 2/66

(54) **Verfahren zur Herstellung von 1,1-Diphenylethanen**

(30) Priorität: 27.03.1997 DE 19713071
(71) Anmelder: BASF AKTIENGESELLSCHAFT, 67056 Ludwigshafen (DE)
(72) Erfinder: Gehrer, Eugen, Dr., 67069 Ludwigshafen (DE); Eller, Karsten, Dr., 67059 Ludwigshafen (DE); Effler, Günther, 67105 Schifferstadt (DE)
(74) Vertreter: Isenbruck, Günter, Dr.

(57) **Zusammenfassung**

In einem Verfahren zur Herstellung von gegebenenfalls substituierten 1,1-Diphenylethanen durch katalytische Umsetzung von Benzol und Styrol in Gegenwart eines Zeolith-Katalysators, wobei Benzol und Styrol unabhängig je 1 bis 5 Substituenten, ausgewählt aus Halogen, C₁₋₁₀-Alkyl, C₁₋₁₀-Alkoxy, C₁₋₁₀-Acyl, C₁₋₁₀-Acyloxy oder Cyano aufweisen können, ist der Zeolith-Katalysator vom Typ EMT oder ZSM-20.

## Beschreibung

Die Erfindung betrifft ein Verfahren zur Herstellung von gegebenenfalls substituierten 1,1-Diphenylethanen und die Verwendung bestimmter Katalysatoren für deren Herstellung.

Diphenylethan ist ein wichtiges Zwischenprodukt für die Herstellung von Diphenylethen, das als Comonomer für die Herstellung von Kunststoffen verwendet wird. Diphenylethen kann durch katalytische Dehydrierung von Diphenylethan in guten Ausbeuten gewonnen werden. Die Herstellung von Diphenylethan ist jedoch nach den bisher üblichen Methoden nicht vollständig befriedigend gelöst.

In der DE-A-195 16 717 ist ein Verfahren zur Herstellung von Diarylethanen aus gegebenenfalls substituiertem Benzol und gegebenenfalls substituiertem Sryrol beschrieben. Die Umsetzung wird dabei in Gegenwart von Beta-Zeolithen oder Mordeniten als Heterogenkatalysatoren durchgeführt. Eine Umsetzung von Styrol und Benzol ist möglich, jedoch muß die stationäre Styrolkonzentration möglichst klein gehalten werden, um eine selektive Alkylierung von Benzol mit Styrol zum 1,1-Diphenylethan zu ermöglichen. Bei höheren stationären Sryrolkorzentrationen steigt der Nebenproduktanteil an. Es wird deshalb üblicherweise mit einem hohen Benzolüberschuß gearbeitet, wodurch gute Ausbeuten erhalten werden, jedoch auch ein erheblicher Aufwand bei der Aufarbeitung und Reingewinnung des 1,1-Diphenylethans erforderlich ist.

Aufgabe der vorliegenden Erfindung ist die Bereitstellung eines Katalysators für die Umsetzung von Benzol und Styrol zu 1,1-Diphenylethan, der gegenüber den bekannten Katalysatoren eine höhere Selektivität aufweist und höhere stationäre Styrolkorzentrationen toleriert, ohne daß ein höherer Anteil an Nebenprodukten erhalten wird.

Erfindungsgemäß wurde gefunden, daß die Aufgabe gelöst wird durch Verwendung von Zeolith-Katalysatoren der Typen ZSM-20 und/oder EMT zur Herstellung des gegebenenfalls substituierten 1,1-Diphenylethans.

Die Katalysatoren liegen dabei vorzugsweise in ihrer H-Form vor.

Der erfindungsgemäß eingesetzte Zeolith-Katalysator vom Typ ZSM-20, sowie Verfahren zu seiner Herstellung sind in der US 3,972,983 beschrieben. Von den in US 3,972,983 aufgeführten Katalysatoren werden insbesondere die H-Formen eingesetzt. Sie sind strukturell Mischkristalle aus EMT-und Y-Zeolithen.

Die ebenfalls erfindungsgemäß einsetzbaren EMT-Zeolithe sind beispielsweise in der EP-A-0 364 352 und der FR-A-2 638 444 beschrieben. Sie leiten sich von den Faujasit-Strukturen ab und unterscheiden sich von diesen durch eine andere Verknüpfungssequenz. Während Faujasite, wie Y-Zeolithe, eine kubische Struktur haben, besitzen EMT-Zeolithe eine hexagonale Struktur. Verfahren zu ihrer Herstellung und verwendbare EMT-Zeolithe sind in der EP-A-0 364 352 beschrieben. Sie lassen sich mit Hilfe eines Templates (18-Krone-6) aus einer Gelmischung mit der Zusammensetzung von beispielsweise 10 SiO₂ : 1 Al₂O₃ : 2,4 Na₂O : 1 18-Krone-6 : 130 H₂O bei Temperaturen im Bereich 80 bis 110°C im Verlaufe von 1 bis 2 Wochen kristallisieren. Zur Beschleunigung der Kristallisation können Additive wie NaF, Oxalsäure, Citronensäure zugesetzt werden. Besonders vorteilhaft werden große Zeolithkristalle mit Durchmessern von mehr als 2 µm eingesetzt, die unter Einsatz von Aerosil (SiO₂) als Siliciumquelle gewonnen werden können. Je nach Kristallisationsbedingungen können neben dem reinen EMT auch ZSM-20-Zeolithe entstehen.

Die erfindungsgemäß eingesetzten Katalysatoren können entweder in Pulverform im Reaktionsgemisch suspendiert werden oder als Formkörper, beispielsweise in einem Festbettreaktor, eingesetzt werden. Dabei können beliebige geeignete Formen, wie Stränge, Tabletten, Pellets, Kugeln, Granulate oder Splitt eingesetzt werden. Verfahren zur Herstellung der Formkörper aus den erfindungsgemaßen Katalysatoren sind bekannt.

Im erfindungsgemäßen Verfahren zur Herstellung von gegebenenfalls substituierten 1,1-Diphenylethanen durch katalytische Umsetzung von Benzol und Sryrol in Gegenwart eines Zeolith-Katalysators vom Typ EMT oder ZSM-20 können Benzol und Styrol unabhängig 1 bis 5 Substituenten ausgewählt aus Halogen, C₁₋₁₀-Alkyl, C₁₋₁₀-Alkoxy, C₁₋₁₀-Acyl, C₁₋₁₀-Acyloxy oder Cyano aufweisen.

Unter dem Begriff "Benzol" werden dabei sowohl das nicht substituierte Benzol als auch substituierte Benzole verstanden. Ebenso werden unter dem Begriff "Styrol" das nicht substituierte Styrol wie auch am Phenylrest substituierte Styrole verstanden. Die Vinylgruppe weist im Styrol oder substituierten Styrol vorzugsweise keine weiteren Substituenten auf. Die Substituenten im Benzol und Styrol liegen in jeder Position unabhängig voneinander vor, d.h. an allen Molekülpositionen können unterschiedliche Substituenten vorliegen. Vorzugsweise weisen das erfindungsgemäß eingesetzte Benzol und Styrol unabhängig 0 bis 3, besonders bevorzugt 0 bis 2, insbesondere 0 bis 1 Substituenten am aromatischen Kern auf. Insbesondere bevorzugt werden Benzol und Styrol in unsubstituierter Form verwendet.

Als Substituenten kommen Halogenatome, wie Fluor-, Chlor-, Brom- oder Iodatome in Betracht. Die Alkyl-, Alkoxy-, Acyl- oder Acyloxy-Substituenten weisen vorzugsweise 1 bis 5, besonders bevorzugt 1 bis 3 C-Atome auf. Vorzugsweise weisen diese Reste lineare aliphatische Kohlenstoffketten auf.

Besonders bevorzugt weisen das eingesetzte Benzol und Sryrol unabhängig maximal je einen Substituenten auf, wobei der Substituent im Styrol in der p-Position angeordnet ist. Dabei kann der Substituent wie vorstehend ausgewählt sein.

Die Umsetzung von Benzol und Styrol wird vorzugsweise in flüssiger Phase durchgeführt. Dabei liegt die Reaktionstemperatur vorzugsweise im Bereich von -20 bis 300°C, besonders bevorzugt 10 bis 250°C, insbesondere 50 bis 200°C. Der Druck beträgt vorzugweise 1 bis 100 bar, besonders bevorzugt ist ein Druck, der gleich oder größer als der Eigendruck des Systems bei der jeweiligen Temperatur ist.

Dabei kann die Umsetzung kontinuierlich oder diskontinuierlich durchgeführt werden.

Die Umsetzung führt mit hoher Selektivität zum gewünschten, gegebenenfalls substituierten 1,1-Diphenylethan. Deshalb können bei der Umsetzung relativ hohe stationäre Styrolkonzentrationen toleriert werden, ohne daß der Nebenproduktanteil bei der Umsetzung zu hoch wird. Vorzugsweise beträgt die stationäre Styrolkonzentration im Reaktionsgemisch maximal 5, besonders bevorzugt maximal 2, insbesondere maximal 0,5, speziell maximal 0,1 Gew.-%, bezogen auf das Reaktionsgemisch ohne Katalysator.

Gemäß einer bevorzugten Ausführungsform der Erfindung legt man den Katalysator zusammen mit dem Benzol vor und führt das Styrol so zu, daß das Styrol laufend abreagieren kann und die stationäre Styrolkonzentration die vorstehenden Maximalwerte nicht übersteigt. Entsprechende Sryrolkonzentrationen können dabei durch die Geschwindigkeit der Zuführung in Abhängigkeit der Umsetzungstemperatur eingestellt werden. Bei dieser diskontinuierlichen Durchführung wird das Gemisch aus Benzol und Katalysator auf die Reaktionstemperatur gebracht und dann mit Styrol versetzt. Anstelle von Styrol kann auch ein Styrol/Benzol-Gemisch zudosiert werden, wobei das zugeführte Styrol laufend abreagiert und seine Konzentration somit gering bleibt.

Bei der kontinuierlichen Verfahrensführung werden vorzugsweise der Katalysator und gegebenenfalls Benzol in einem kontinuierlich betriebenen Rührkessel oder Autoklaven vorgelegt und ein Styrol/Benzol-Gemisch so zugeführt, daß die stationäre Styrolkonzentration die vorstehenden Maximalwerte nicht übersteigt. Wiederum kann die Konzentration über die Geschwindigkeit der Zuführung von Styrol oder Styrol/Benzol-Gemisch eingestellt werden. Der Rührkessel ist dabei mit einem Überlauf mit Druckhalteventil ausgestattet. Der Katalysator wird als Suspension im Berzol vorgelegt oder in Form der vorstehend aufgeführten Formkörper eingesetzt. Zum Anfahren wird der Reaktor mit Benzol gefüllt und auf Reaktionstemperatur gebracht. Anschließend wird kontinuierlich Styrol oder vorzugsweise ein Styrol/Benzol-Gemisch so zudosiert, daß sich die niedrige stationäre Sryrolkonzentration einstellt. Das am Überlauf austretende Reaktionsgemisch kann durch einen Settler oder ein Filter geleitet werden, um vom Katalysator getrennt zu werden. Der Katalysator kann auch in Form von Formkörpern eingebaut werden, so daß eine Abtrennung nicht nötig ist.

Weiterhin kann man den Katalysator als Formkörper in einer Reaktivkolonne einsetzen, ein Styrol/Benzol-Gemisch kontinuierlich oder diskontinuierlich zuführen und das gebildete, gegebenenfalls substituierte 1,1-Diphenylethan über den Sumpf der Kolonne abziehen. Dabei wird nicht umgesetztes Styrol/Benzol-Gemisch über den Kopf der Kolonne abgezogen und kann der Kolonne wieder zugeführt werden.

Weiterhin kann der Katalysator auch in einem Festbettreaktor, insbesondere Rohrreaktor vorgelegt werden, wobei mehr als 50, vorzugsweise mehr als 70, insbesondere mehr als 90 Gew.-% des Produktaustrages zur Rückdurchmischung in den Reaktoreingang zurückgeführt werden. Somit wird ein großer Teil des Produktaustrages zum Verdünnen des Eduktstromes wieder zurückgeführt Der Rohrreaktor wird kontinuierlich von einem Styrol/Benzol-Gemisch durchströmt.

Die Erfindung wird nachstehend anhand von Beispielen näher erläutert.

### Beispiele:

### Herstellung des Katalysators

### Beispiel 1

lösung 1 : 5,1 g Al₂O₃ (frisch gefälltes Al(OH)₃ aus Al(NO₃)_{3*}8 H₂O und 25%igem NH₃/H₂O) wurden in einer lösung von 9,6 g NaOH und 80 g Wasser gelöst.
Lösung 2 : 12,8 g Kronenether 18-Krone-6 (von Merck-Schuchardt) wurden in 75 g Kieselsol (Ludox® AS 40 Von DuPont, = 40% SiO₂ in Wasser gelöst).
Lösung 1 und Lösung 2 wurden vereinigt und 10 Minuten bei Raumtemperatur gerührt. Es entstand ein Gel, das 3 Tage bei Raumtemperatur gealtert wurde. Anschließend wurde das Produkt im Autoklaven 9 Tage lang bei 100°C und 7 Tage bei 110°C zur Kristallisation gebracht. Zur Aufarbeitung wurde das Produkt abfiltriert, mit Wasser neutral gewaschen, bei 110°C getrocknet und anschließend 20 h lang bei 550°C calciniert. Die Ausbeute betrug 26 g. Das Produkt wurde viermal mit 10%iger NH₄Cl-Lösung ausgetauscht, Cl-frei gewaschen, bei 110°C getrocknet und 3 h bei 550°C calciniert.

Der Katalysator hatte eine BET-Oberfläche von 701 m²/g und das Röntgenbeugungsspektrum stimmte mit dem Spektrum vom Typ ZSM-20 überein. Die Elementaranalyse ergab 8,6% Al, 33,0% Si und 0,07% Na.

### Beispiel 2

13 g Katalysator aus dem Beispiel 1 wurden mit 3,25 g Pural® SB (von Condea) und einer lösung von 0,25 g Ameisensäure in 19 g Wasser 30 Minuten lang im Kneter verdichtet. Diese Masse wurde anschließend zu 2 mm Strängen gepreßt und nach dem Trocknen bei 110°C 16 h lang bei 500°C calciniert.

### Beispiel 3

Lösung 1 : 4,08 g Al₂O₃ (frisch gefälltes Al(OH)₃ aus Al(NO₃)₃*8 H₂O und 25%igem NH₃/H₂O) wurden in einer Lösung von 7,68 g NaOH und 80 g Wasser gelöst.
Lösung 2 : 10,24 g 18-Krone-6 wurden in 60 g Wasser gelöst und mit 24 g Aerosil 200 (SiO₂ von Degussa) verrührt.
Lösung 1 und Lösung 2 wurden vereinigt und 10 Minuten bei Raumtemperatur gerührt. Es entstand ein Gel, das 3 Tage bei Raumtemperatur gealtert wurde. Anschließend wurde das Produkt im Autoklaven 16 Tage lang bei 120°C zur Kristallisation gebracht. Zur Aufarbeitung wurde das Produkt abfiltriert, mit Wasser neutral gewaschen, bei 110°C getrocknet und anschließend 20 h lang bei 550°C calciniert. Die Ausbeute betrug 21 g. Das Produkt wurde viermal mit 10%iger NH₄Cl-Lösung ausgetauscht, Cl-frei gewaschen, bei 110°C getrocknet und 3 h bei 550°C calciniert.

### Durchführung der katalytischen Umsetzungen

Zur Analyse der nachfolgenden Experimente wurde ein Gaschromatograph mit einer 30 m langen Kapillarsäule (DB5, von J & W Scientific (Fisions), 0.1 um) verwendet. (Temperaturprogramm: 5 Minuten bei 60°C, 10°C/Minute bis 300°C, 15 Minuten Endtemperatur). Die Quantifizierung erfolgte mit Hilfe von Toluol als internen Standard.
Die 3 möglichen Styroldimere (Identifizierung mittels GC/MS) wurden in der folgenden Zusammenstellung zusammengezählt. Ihr Eichfaktor wurde mit dem für Diphenylethan gleichgesetzt. Die Eichfaktoren für Benzol (nicht aufgelistet), Styrol und Diphenylethan wurden über eine Konzentrationsreihe mit Toluol als internem Standard bestimmt.
Als Vergleichsbeispiele wurden Zeolithe vom Typ H-Mordenit (PQ 900 H, der PQ), H-Y (der BASF), H-ZSM-5 (der Degussa) und H-Beta-Zeolith (Zeocat® PB, von Uetikon) eingesetzt.

### Beispiele 4-9

In einem 50 ml Druckglasgefäß wurden 1 g der nachstehend aufgeführten Katalysatoren und 10 ml Styrol/Benzol-Lösung 3 h bei 50°C gerührt. Das Reaktionsprodukt wurde anschließend mittels GC analysiert.

**Tabelle 1**

| **Produktzusammensetzung** | | | | | | |
|---|---|---|---|---|---|---|
| Nr. | Katalysator | Styrol | 1,1-Diphenylethan | Dimere | Trimere | Ausbeute |
| 4 | ZSM-5 | 2,26% | 0,0% | 2,42% | 0,05% | 0,0% |
| 5 | β-Zeolith | 0,0% | 1,77% | 4,85% | 0,2% | 10,1% |
| 6 | Mordenit | 0,0% | 1,59% | 3,27% | 0,1% | 6,1% |
| 7 | H-Y-Zeolith | 0,0% | 1,22% | 2,92% | 0,49% | 6,9% |
| 8 | Beispiel 1 | 0,0% | 3,02% | 3,41% | 0,24% | 17,3% |
| 9 | Beispiel 3 | 0,0% | 4,97% | 3,10% | 0,77% | 28,4% |

Aus den Ergebnissen der Tabelle 1 geht hervor, daß die erfindungsgemäßen Katalysatoren mit gegenüber den Vergleichskatalysatoren wesentlich höherer Ausbeute und wesentlich besserer Selektivität zu 1,1-Diphenylethan führen. Sie sind den für diese Anwendung bekannten Zeolithen überlegen.

### Beispiel 10 (Kontinuierliche Fahrweise)

12,4 g Katalysator aus Beispiel 2 wurden in einen 50 ml fassenden Autoklaven in einem Katalysatorkorb vorgelegt. Der Autoklav wurde mit Benzol gefüllt und auf 200°C aufgeheizt. Anschließend wurde kontinuierlich eine Lösung von 5% Styrol in 95% Benzol mit einer Rate von 120 ml/h zudosiert. Der Überlauf wurde über ein Druckhalteventil (50 bar) ausgetragen und in regelmäßigen Abständen analysiert.

**Tabelle 2**

| **Produktzusammensetzung** | | | | | |
|---|---|---|---|---|---|
| Laufzeit [h] | Styrol | Diphenylethan | Dimere | Trimere | Ausbeute |
| 10 h | 0,13% | 7,3% | 0,04% | 0,30% | 81,8% |
| 18 h | 0,22% | 7,23% | 0,10% | 0,46% | 80,8% |
| 30 h | 0,64% | 5,56% | 0,64% | 0,31% | 59,5% |

## Patentansprüche

1. Verfahren zur Herstellung von gegebenenfalls substituierten 1,1-Diphenylethanen durch katalytische Umsetzung von Benzol und Styrol in Gegenwart eines Zeolith-Katalysators, wobei Benzol und Styrol unabhängig je 1 bis 5 Substituenten, ausgewählt aus Halogen, C₁₋₁₀-Alkyl, C₁₋₁₀-Alkoxy, C₁₋₁₀-Acyl, C₁₋₁₀-Acyloxy oder Cyano aufweisen können, dadurch gekennzeichnet, daß der Zeolith-Katalysator von Typ EMT oder ZSM-20 ist.

2. Verfahren nach Anspruch 1, dadurch gekennzeichnet, daß die Umsetzung bei Temperaturen von -20 bis 300°C und Drücken von 1 bis 100 bar durchgeführt wird.

3. Verfahren nach Anspruch 1 oder 2, dadurch gekennzeichnet, daß im Reaktionsgemisch die stationäre Styrolkonzentration maximal 5 Gew.-%, bezogen auf das gesamte Reaktionsgemisch, beträgt.

4. Verfahren nach einem der Ansprüche 1 bis 3, dadurch gekennzeichnet, daß eingesetztes Benzol und Styrol unabhängig maximal je einen Substituenten am aromatischen Kern aufweisen, wobei der Substituent im Styrol in der p-Position angeordnet ist.

5. Verfahren nach Anspruch 4, dadurch gekennzeichnet, daß unsubstituiertes Benzol und unsubstituiertes Styrol umgesetzt werden.

6. Verfahren nach einem der Ansprüche 1 bis 5, dadurch gekennzeichnet, daß man den Katalysator zusammen mit dem Benzol vorlegt und das Styrol so zuführt, daß die stationäre Styrolkonzentration maximal 5 Gew.-% , bezogen auf das gesamte Reaktionsgemisch, beträgt.

7. Verfahren nach einem der Ansprüche 1 bis 5, dadurch gekennzeichnet, daß man den Katalysator und gegebenenfalls das Benzol in einem kontinuierlich betriebenen Rührkessel oder Autoklaven vorlegt und Styrol oder ein Styrol/Benzol-Gemisch so zuführt, daß die stationäre Sryrolkonzentration maximal 5 Gew.-%, bezogen auf das gesamte Reaktionsgemisch, beträgt.

8. Verfahren nach einem der Ansprüche 1 bis 5, dadurch gekennzeichnet, daß man den Katalysator als Formkörper in einer Reaktivkolonne einsetzt, ein Styrol/Benzol-Gemisch kontinuierlich oder diskontinuierlich zuführt und das gebildete, gegebenenfalls substituierte 1,1-Diphenylethan über den Sumpf der Kolonne abzieht.

9. Verfahren nach einem der Ansprüche 1 bis 5, dadurch gekennzeichnet, daß man den Katalysator in einem Festbettreaktor vorlegt und mehr als 50 Gew.-% des Produktaustrages zur Rückdurchmischung in den Reaktoreingang zurückführt.

10. Verwendung von Zeolith-Katalysatoren der Typen ZSM-20 und/oder EMT zur Herstellung von gegebenenfalls substituierten 1,1-Diphenylethanen.
